# EUROPEAN PATENT APPLICATION

(11) **EP 1 714 652 A2**
(43) Date of publication of application: **25.10.2006**
(21) Application number: 05027984.3
(22) Date of filing: 14.09.1994
(51) Int. Cl.: A61K 31/426

(54) **Use of thiazolidinediones to prevent or delay onset of niddm**

(30) Priority: 15.09.1993 US 122251; 23.08.1994 US 293899
(62) Divisional of application: 94929204.9
(71) Applicant: Sankyo Company, Limited, Chuo-ku Tokyo 104 (JP)
(72) Inventor: Olefsky, Jerrold, Solano Beach 92075 California (US); Antonucci, Tammy, Mequon 53092 Wisconsin (US); Lockwood, Dean, Ann Arbor 48103 Michigan (US); Norris, Rebecca, Kewadin 49648 Michigan (US)
(74) Representative: Gibson, Christian John Robert

(57) **Abstract**

A novel method of using thiazolidinedione derivatives and related antihyperglycemic agents to treat populations experiencing impaired glucose tolerance in order to prevent or delay the onset of noninsulin-dependent diabetes mellitus (NIDDM) and complications arising therefrom is disclosed.

## Description

### FIELD OF THE INVENTION

The present invention pertains to a number of compounds which can be used to treat impaired glucose intolerance in order to prevent or delay the onset of noninsulin-dependent diabetes mellitus (NIDDM). More specifically, the present invention involves in one embodiment administering to a patient certain known thiazolidinedione derivatives and related antihyperglycemic agents which reduce fasting insulin levels and return normal glucose tolerance to an individual, thus preventing or delaying the onset of NIDDM or complications resulting therefrom.

### BACKGROUND

Diabetes is one of the most prevalent chronic disorders worldwide with significant personal and financial costs for patients and their families, as well as for society. Different types of diabetes exist with distinct etiologies and pathogeneses. For example, diabetes mellitus is a disorder of carbohydrate metabolism, characterized by hyperglycemia and glycosuria and resulting from inadequate production or utilization of insulin.

Diabetes mellitus often develops from certain at risk populations, one such population is individuals with impaired glucose tolerance (IGT). Impaired glucose tolerance is a condition intermediate between frank, noninsulin-dependent diabetes mellitus and normal glucose tolerance in which the affected person's postprandial glucose response is abnormal as assessed by 2-hour postprandial plasma glucose levels. This IGT population progresses to a certain form of diabetes mellitus, specifically noninsulin-dependent diabetes mellitus (NIDDM).

NIDDM or otherwise referred to as Type II diabetes is the form of diabetes mellitus which occurs predominantly in adults in whom adequate production of insulin is available for use, yet a defect exists in insulin-mediated utilization and metabolism of glucose in peripheral tissues. It has been shown that for some people with diabetes a genetic predisposition results in a mutation in the gene(s) coding for insulin and/or the insulin receptor and/or insulin-mediated signal transduction factor(s), thereby resulting in ineffective insulin and/or insulin-mediated effects thus impairing the utilization or metabolism of glucose. The population with impaired glucose tolerance progresses to NIDDM at a rate of 5% to 10% of cases per year.

Failure to treat NIDDM can result in mortality due to cardiovascular disease and in other diabetic complications including retinopathy, nephropathy, and peripheral neuropathy. For many years treatment of NIDDM has involved a program aimed at lowering blood sugar with a combination of diet and exercise. Alternatively, treatment of NIDDM involved oral hypoglycemic agents, such as sulfonylureas alone or in combination with insulin injections. Recently, alpha-glucosidase inhibitors, such as acarbose, have been shown to be effective in reducing the postprandial rise in blood glucose (Lefevre, et al., Drugs 1992;44: 29-38). In Europe and Canada another treatment used primarily in obese diabetics is metformin, a biguanide.

In any event, what is required is a method of treating populations experiencing impaired glucose tolerance in order to prevent or delay the onset of NIDDM thereby bringing relief of symptoms, improving the quality of life, preventing acute and long-term complications, reducing mortality and treating accompanying disorders of those at risk for NIDDM. The methods of using the disclosed compounds for treating populations experiencing impaired glucose tolerance to prevent or delay the onset of NIDDM as taught herein meet these objectives.

Compounds useful for practicing the present invention, and methods of making these compounds are known. Some of these compounds are disclosed in WO 91/07107; WO 92/02520; WO 94/01433; WO 89/08651; JP Kokai 69383/92; U.S. Patent Nos. 4,287,200; 4,340,605; 4,438,141; 4,444,779; 4,461,902; 4,572,912; 4,687,777; 4,703,052; 4,725,610; 4,873,255; 4,897,393; 4,897,405; 4,918,091; 4,948,900; 5,002,953; 5,061,717; 5,120,754; 5,132,317; 5,194,443; 5,223,522; 5,232,925; and 5,260,445. The active compounds disclosed in these publications are useful as therapeutic agents for the treatment of diabetes, hyperglycemia, hypercholesterolemia, and hyperlipidemia. The disclosure of these publications are incorporated herein by reference in particular with respect to the active compounds disclosed therein, and methods of preparation thereof. These compounds are useful for the treatment of impaired glucose tolerance (IGI) in order to prevent or delay onset of NIDDM and complications resulting therefrom, in accordance with the present invention.

There is no disclosure in the above-identified references to use the compounds identified in this present application in the treatment of populations experiencing impaired glucose tolerance in order to prevent or delay the onset of NIDDM and complications resulting therefrom.

### SUMMARY OF THE INVENTION

The present invention provides a method for the treatment of impaired glucose tolerance in order to prevent or delay the onset of NIDDM. It is known that persons with impaired glucose tolerance have a much higher rate of progression to NIDDM than persons with normal glucose tolerance. Saad, et al., New Engl J Med 1988; 319:1500-6. If impaired glucose tolerance can be normalized, it is likely that the progression to NIDDM will be delayed or prevented in this population.

Compounds useful for practicing the present invention reduce fasting insulin levels, improve insulin sensitivity, and return glucose tolerance to the normal range for many individuals. As agents having the aforementioned effects (in the return of glucose tolerance), the compounds of the following formulas are useful in prophylactically treating individuals to prevent or delay the onset of NIDDM.

Accordingly, the present invention is the use of compounds of Formula I 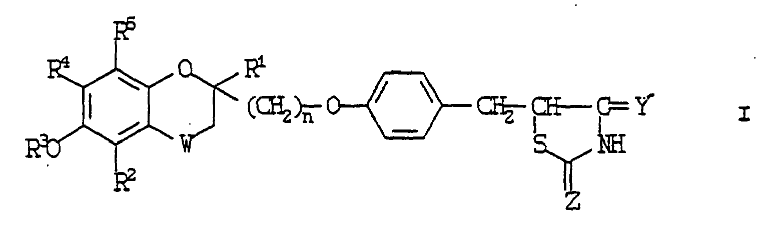 wherein R¹ and R² are the same or different and each represents a hydrogen atom or a C₁-C₅ alkyl group;
R³ represents a hydrogen atom, a C₁-C₆ aliphatic acyl group, an alicyclic acyl group, an aromatic acyl group, a heterocyclic acyl group, an araliphatic acyl group, a (C₁-C₈ alkoxy)carbonyl group, or an aralkyloxycarbonyl group;
R⁴ and R⁵ are the same or different and each represents a hydrogen atom, a C₁-C₅ alkyl group or a C₁-C₅ alkoxy group, or R⁴ and R⁵ together represent a C₁-C₄ alkylenedioxy group;
n is 1, 2, or 3;
W represents the -CH₂-, >CO, or CH-OR⁶ group (in which R⁶ represents any one of the atoms or groups defined for R³ and may be the same as or different from R³); and
Y and Z are the same or different and each represents an oxygen atom or an imino (=NH) group;
and pharmaceutically acceptable salts thereof.

The present invention is also the use of compounds of the Formula II 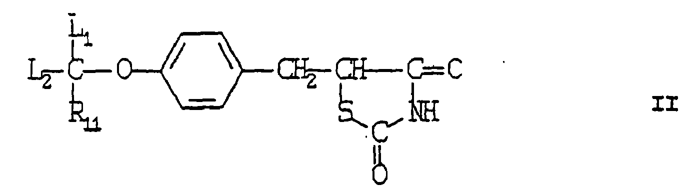 wherein R₁₁ is substituted or unsubstituted alkyl, alkoxy, cycloalkyl, phenylalkyl, phenyl, aromatic acyl group, a 5- or 6-membered heterocyclic group including 1 or 2 heteroatoms selected from the group consisting of nitrogen, oxygen, and sulfur, or a group of the formula 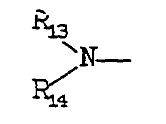 wherein R₁₃ and R₁₄ are the same or different and each is lower alkyl or R₁₃ and R₁₄ are combined to each other either directly or as interrupted by a heteroatom selected from the group consisting of nitrogen, oxygen, and sulfur to form a 5- or 6-membered ring;
wherein R₁₂ means a bond or a lower alkylene group; and
wherein L₁ and L₂ are the same or different and each is hydrogen or lower alkyl or L₁ and L₂ are combined to form an alkylene group; or a pharmaceutically acceptable salt thereof.

The present invention is also the use of compounds of the Formula III 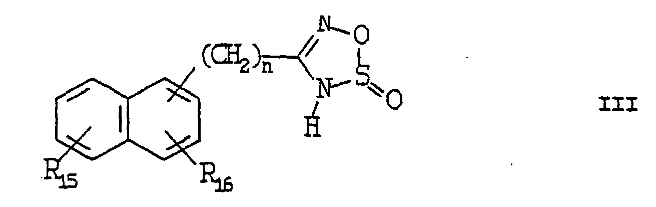 wherein R₁₅ and R₁₆ are independently hydrogen, lower alkyl containing 1 to 6 carbon atoms, alkoxy containing 1 to 6 carbon atoms, halogen, ethynyl, nitrile, methylthio, trifluoromethyl, vinyl, nitro, or halogen substituted benzyloxy; n is 0 to 4 and the pharmaceutically acceptable salts thereof.

The present invention is also directed to the use of compounds of the Formula IV 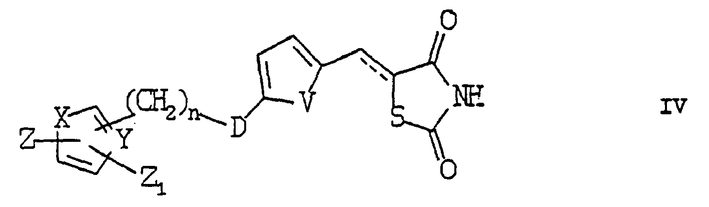 wherein the dotted line represents a bond or no bond;
V is -CH = CH-, -N = CH-, -CH = N- or S;
D is CH₂, CHOH, CO, C = NOR₁₇ or CH = CH;
X is S, O, NR₁₈, -CH = N or -N = CH;
Y is CH or N;
Z is hydrogen, (C₁-C₇) alkyl, (C₃-C₇)cycloalkyl, phenyl, naphthyl, pyridyl, furyl, thienyl, or phenyl mono- or disubstituted with the same or different groups which are (C₁-C₃)alkyl, trifluoromethyl, (C₁-C₃)alkoxy, fluoro, chloro, or bromo;
Z₁ is hydrogen or (C₁-C₃)alkyl;
R₁₇ and R₁₈ are each independently hydrogen or methyl; and
n is 1, 2, or 3;
the pharmaceutically acceptable cationic salts thereof;
and the pharmaceutically acceptable acid addition salts thereof when the compound contains a basic nitrogen.

The present invention is also directed to the use of compounds of the Formula V 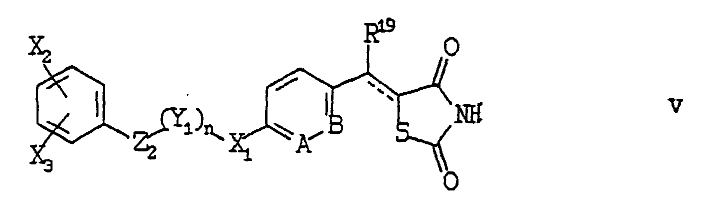 wherein the dotted line represents a bond or no bond;
A and B are each independently CH or N, with the proviso that when A or B is N, the other is CH;
X₁ is S, SO, SO₂, CH₂, CHOH, or CO;
n is 0 or 1;
Y₁ is CHR₂₀ or R₂₁, with the proviso that when n is 1 and
Y₁ is NR₂₁, X₁ is SO₂ or CO;
Z₂ is CHR₂₂, CH₂CH₂, CH=CH, 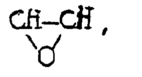 OCH₂, SCH₂, SOCH₂ or SO₂CH₂;
R₁₉, R₂₀, R₂₁, and R₂₂ are each independently hydrogen or methyl; and
X₂ and X₃ are each independently hydrogen, methyl, trifluoromethyl, phenyl, benzyl, hydroxy, methoxy, phenoxy, benzyloxy, bromo, chloro, or fluoro;
a pharmaceutically acceptable cationic salt thereof; or
a pharmaceutically acceptable acid addition salt thereof when A or B is N.

The present invention also relates to the use of compounds of the Formula VI 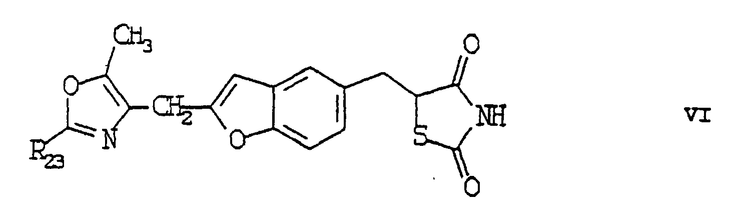 or a pharmaceutically acceptable salt thereof wherein R₂₃ is alkyl of 1 to 6 carbon atoms, cycloalkyl of 3 to 7 carbon atoms, phenyl or mono- or di-substituted phenyl wherein said substituents are independently alkyl of 1 to 6 carbon atoms, alkoxy of 1 to 3 carbon atoms, halogen, or trifluoromethyl.

The present invention also provides the use of a compound of Formula VII 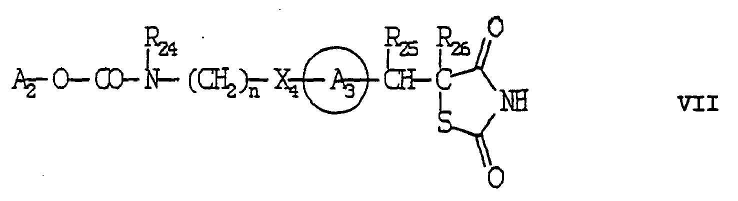 or a tautomeric form thereof and/or a pharmaceutically acceptable salt thereof, and/or a pharmaceutically acceptable solvate thereof, wherein:
A₂ represents an alkyl group, a substituted or unsubstituted aryl group, or an aralkyl group wherein the alkylene or the aryl moiety may be substituted or unsubstituted;
A₃ represents a benzene ring having in total up to 3 optional substituents;
R₂₄ represents a hydrogen atom, an alkyl group, an acyl group, an aralkyl group wherein the alkyl or the aryl moiety may be substituted or unsubstituted, or a substituted or unsubstituted aryl group; or A₂ together with R₂₄ represents substituted or unsubstituted C₂₋₃ polymethylene group, optional substituents for the polymethylene group being selected from alkyl or aryl or adjacent substituents together with the methylene carbon atoms to which they are attached form a substituted or unsubstituted phenylene group;
R₂₅ and R₂₆ each represent hydrogen, or R₂₅ and R₂₆ together represent a bond;
X₄ represents O or S; and
n represents an integer in the range of from 2 to 6.

The present invention also provides the use of a compound of Formula VIII 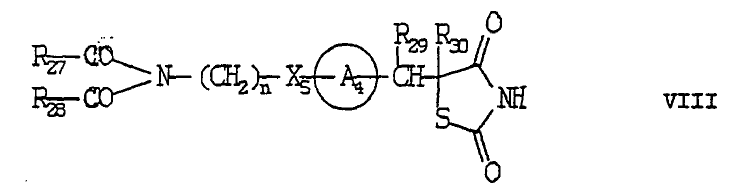 or a tautomeric form thereof and/or a pharmaceutically acceptable salt thereof, and/or a pharmaceutically acceptable solvate therefor, wherein:
R₂₇ and R₂₈ each independently represent an alkyl group, a substituted or unsubstituted aryl group, or an aralkyl group being substituted or unsubstituted in the aryl or alkyl moiety; or R₂₇ together with R₂₈ represents a linking group, the linking group consisting of an optionally substituted methylene group and either a further optionally substituted methylene group or an O or S atom, optional substituents for the said methylene groups being selected from alkyl-, aryl, or aralkyl, or substituents of adjacent methylene groups together with the carbon atoms to which they are attached form a substituted or unsubstituted phenylene group;
R₂₉ and R₃₀ each represent hydrogen, or R₂₉ and R₃₀ together represent a bond;
A₄ represents a benzene ring having in total up to 3 optional substituents;
X₅ represents O or S; and
n represents an integer in the range of from 2 to 6.

The present invention also provides the use of a compound of Formula IX 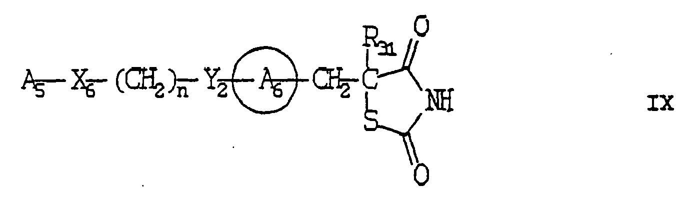 or a tautomeric form thereof and/or a pharmaceutically acceptable salt thereof, and/or a pharmaceutically acceptable solvate thereof, wherein:
A₅ represents a substituted or unsubstituted aromatic heterocyclyl group;
A₆ represents a benzene ring having in total up to 5 substituents;
X₆ represents O, S, or NR₃₂ wherein R₃₂ represents a hydrogen atom, an alkyl group, an acyl group, an aralkyl group, wherein the aryl moiety may be substituted or unsubstituted, or a substituted or unsubstituted aryl group;
Y₂ represents O or S;
R₃₁ represents an alkyl, aralkyl, or aryl group; and
n represents an integer in the range of from 2 to 6.

Suitable aromatic heterocyclyl groups include substituted or unsubstituted, single or fused ring aromatic heterocyclyl groups comprising up to 4 hetero atoms in each ring selected from oxygen, sulphur, or nitrogen.

Favored aromatic heterocyclyl groups include substituted or unsubstituted single ring aromatic heterocyclyl groups having 4 to 7 ring atoms, preferably 5 or 6 ring atoms.

In particular, the aromatic heterocyclyl group comprises 1, 2, or 3 heteroatoms, especially 1 or 2, selected from oxygen, sulphur, or nitrogen.

Suitable values for A₅ when it represents a 5-membered aromatic heterocyclyl group include thiazolyl and oxazoyl, especially oxazoyl.

Suitable values for A₅ when it represents a 6-membered aromatic heterocyclyl group include pyridyl or pyrimidinyl.

Suitable R₃₁ represents an alkyl group, in particular a C₁₋₆ alkyl group, for example a methyl group.
Preferably, A₅ represents a moiety of formula (a), (b), or (c): 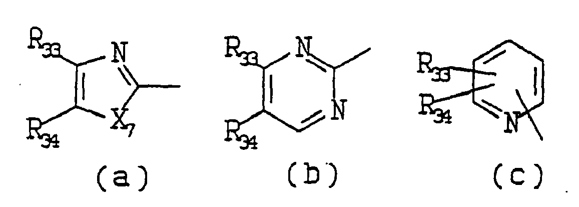 wherein:
R₃₃ and R₃₄ each independently represents a hydrogen atom, an alkyl group, or a substituted or unsubstituted aryl group or when R₃₃ and R₃₄ are each attached to adjacent carbon atoms, then R₃₃ and R₃₄ together with the carbon atoms to which they are attached form a benzene ring wherein each carbon atom represented by R₃₃ and R₃₄ together may be substituted or unsubstituted; and in the moiety of Formula (a), X₇ represents oxygen or sulphur.

In one favored aspect R₃₃ and R₃₄ together represent a moiety of Formula (d): 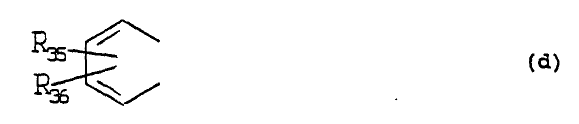 wherein R₃₅ and R₃₆ each independently represent hydrogen, halogen, substituted or unsubstituted alkyl, or alkoxy.

The present invention also provides for the use of compounds for Formula X 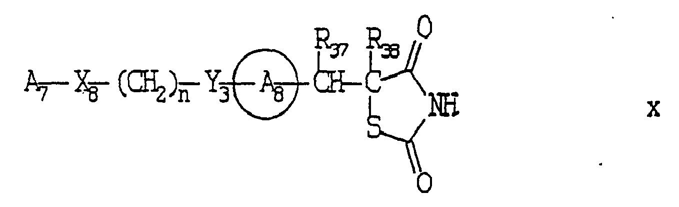 or a tautomeric form thereof and/or a pharmaceutically acceptable salt thereof, and/or a pharmaceutically acceptable solvate thereof, wherein:
A₇ represents a substituted or unsubstituted aryl group;
A₈ represents a benzene ring having in total up to 5 substituents;
X₈ represents O, S, or NR₃₉ wherein R₃₉ represents a hydrogen atom, an alkyl group, an acyl group, an aralkyl group, wherein the aryl moiety may be substituted or unsubstituted, or a substituted or unsubstituted aryl group;
Y₃ represents O or S;
R₃₇ represents hydrogen;
R₃₈ represents hydrogen or an alkyl, aralkyl, or aryl group or R₃₇ together with R₃₈ represents a bond; and
n represents an integer in the range of from 2 to 6.

The present invention is also directed to the use of compounds of the Formula 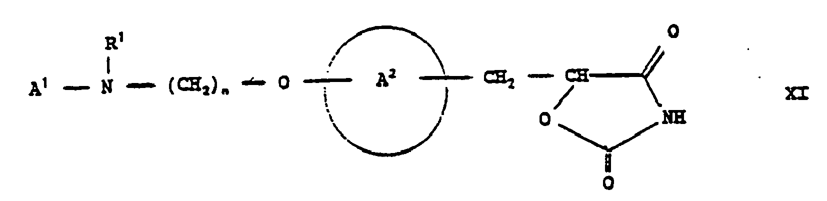 or a tautomeric form thereof and/or a pharmaceutically acceptable salt thereof, and/or a pharmaceutically acceptable solvate thereof, wherein:
A¹ represents a substituted or unsubstituted aromatic heterocyclyl group;
R¹ represents a hydrogen atom, an alkyl group, an acyl group, an aralkyl group, wherein the aryl moiety may be substituted or unsubstituted, or a substituted or unsubstituted aryl group;
A² represents a benzene ring having in total up to five substituents; and
n represents an integer in the range of from 2 to 6.

Suitable aromatic heterocyclyl groups include substituted or unsubstituted, single or fused ring aromatic heterocyclyl groups comprising up to 4 hetero atoms in each ring selected from oxygen, sulphur or nitrogen.

Favoured aromatic heterocyclyl groups include substituted or unsubstituted single ring aromatic heterocyclyl groups having 4 to 7 ring atoms, preferably 5 or 6 ring atoms.

In particular, the aromatic heterocyclyl group comprises 1, 2 or 3 heteroatoms, especially 1 or 2, selected from oxygen, sulphur or nitrogen.

Suitable values for A¹ when it represents 5-membered aromatic heterocyclyl group include thiazolyl and oxazolyl, especially oxazolyl.

Suitable values for A¹ when it represents a 6-membered aromatic heterocyclyl group include pyridyl or pyrimidinyl.

Preferably, A¹ represents a moiety of formula (a), (b) or (c): 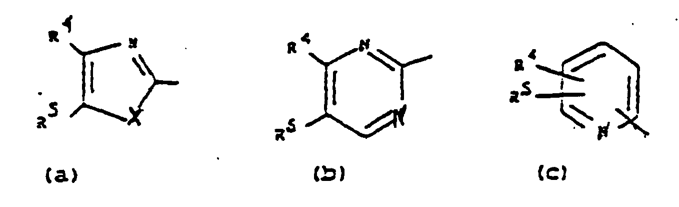 wherein:
R⁴ and R⁶ each independently represents a hydrogen atom, an alkyl group or a substituted or unsubstituted aryl group or when R⁴ and R⁵ are each attached to adjacent carbon atoms, then R⁴ and R⁵ together with the carbon atoms to which they are attached form a benzene ring wherein each carbon atom represented by R⁴ and R⁵ together may be substituted or unsubstituted; and in the moiety of formula (a)
X represents oxygen or sulphur.

The present invention is also directed to the use of compounds of the Formulas 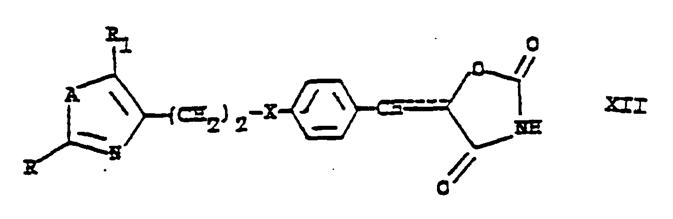 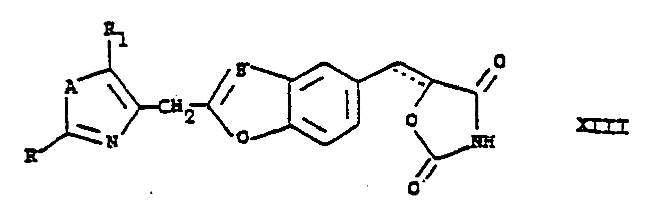 or a pharmaceutically acceptable salt thereof wherein the dotted line represents a bond or no bond; R is cycloalkyl of three to seven carbon atoms, naphthyl, thienyl, furyl, phenyl or substituted phenyl wherein said substituent is alkyl of one to three carbon atoms, alkoxy of one to three carbon atoms, trifluoromethyl, chloro, fluoro or bis(trifluoromethyl); R₁ is alkyl of one to three carbon atoms; X is O or C=O; a is O or S; and B is N or CH.

A preferred group of compounds are those of formula XI wherein the dotted line represents no bond, R₁ is methyl, X is O and A is O. Especially preferred within this group are the compounds where R is phenyl, 2-naphthyl and 3,5-bis(trifluoromethyl)phenyl.

A second group of preferred compounds are those of formula XII wherein the dotted line represents no bond, R₁ is methyl and A is O. Especially preferred within this group are compounds where B is CH and R is phenyl, p-tolyl, m-tolyl, cyclohexyl and 2-naphthyl. Also especially preferred is the compound where B is N and R is phenyl.

A still further embodiment of the present invention is the use of pharmaceutical composition for administering an effective amount of a compound of the preceding Formulas I through XIII along with a pharmaceutically acceptable carrier in unit dosage form in the treatment methods mentioned above.

### DETAILED DESCRIPTION OF THE PREFERRED EMBODIMENTS

The compounds used in the treatment methods of the invention, which are 5-[4-(chromoanalkoxy)benzyl]-thiazolidene derivatives, may be represented by the Formulas (Ia), (Ib), and (Ic) 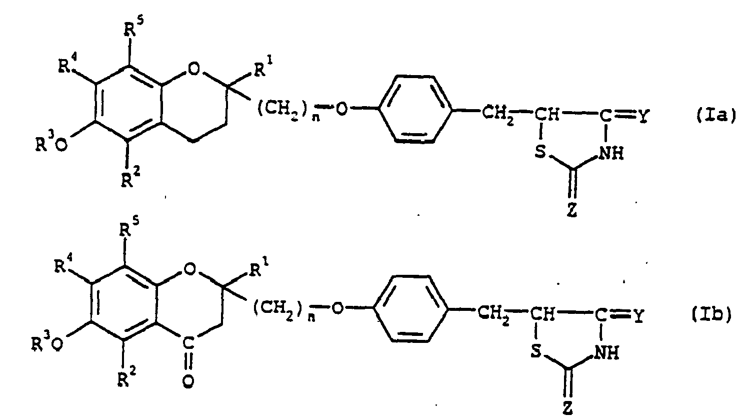 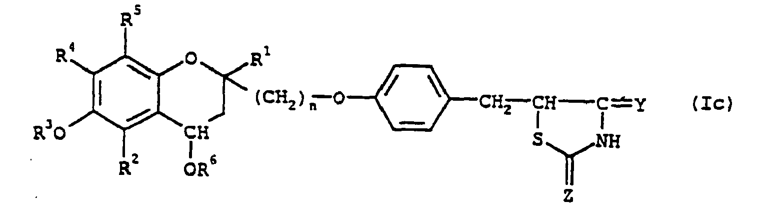 (in which R¹, R², R³, R⁴, R⁵, R⁶, n, Y, and Z are as defined above) and include pharmaceutically acceptable salts thereof.

In the compounds of the invention, where R¹ or R² represents an alkyl group, this may be a straight or branched chain alkyl group having from 1 to 5 carbon atoms and is preferably a primary or secondary alkyl group, for example the methyl, ethyl, propyl, isopropyl, butyl, isobutyl, pentyl, or isopentyl group.

Where R³, R⁶, or R^{6'} represents an aliphatic acyl group,'this preferably has from 1 to 6 carbon atoms and may include one or more carbon-carbon double or triple bonds. Examples of such groups include the formyl, acetyl, propionyl, butyryl, isobutyryl, pivaloyl, hexanoyl, acryloyl, methacryloyl, and crotonyl groups.

Where R³, R⁶, or R^{6'} represents an alicyclic acyl group, it is preferably a cyclopentanecarbonyl, cyclohexanecarbonyl, or cycloheptanecarbonyl group.

Where R³, R⁶, or R^{6'} represents an aromatic acyl group, the aromatic moiety thereof may optionally have one or more substituents (for example, nitro, amino, alkylamino, dialkylamino, alkoxy, halo, alkyl, or hydroxy substituents); examples of such aromatic acyl groups included the benzoyl, p-nitrobenzoyl, m-fluorobenzoyl, o-chlorobenzoyl, p-aminobenzoyl, m-(dimethylamino)-benzoyl, o-methoxybenzoyl, 3,4-dichlorobenzoyl, 3,5-dit-butyl-4-hydroxybenzoyl, and 1-naphthoyl groups.

Where R³, R⁶, or R^{6'} represents a heterocyclic acyl group, the heterocyclic moiety thereof preferably has one or more, preferably one, oxygen, sulfur, or nitrogen hetero atoms and has from 4 to 7 ring atoms; examples of such heterocyclic acyl groups include the 2-furoyl, 3-thenoyl, 3-pyridinecarbonyl (nicotinoyl), and 4-pyridinecarbonyl groups.

Where R³, R⁶, or R^{6'} represents an araliphatic acyl group, the aliphatic moiety thereof may optionally have one or more carbon-carbon double or triple bonds and the aryl moiety thereof may optionally have one or more substituents (for example, nitro, amino, alkylamino, dialkylamino, alkoxy, halo, alkyl, or hydroxy substituents); examples of such araliphatic acyl groups include the phenylacetyl, p-chlorophenylacetyl, phenylpropionyl, and cinnamoyl groups.

Where R³, R⁶, or R^{6'} represents a (C₁-C₆ alkoxy)carbonyl group, the alkyl moiety thereof may be any one of those alkyl groups as def ined for R¹ and R², but is preferably a methyl or ethyl group, and the alkoxycarbonyl group represented by R³, R⁶, or R^{6'} is therefore preferably a methoxycarbonyl or ethoxycarbonyl group.

Where R³, R⁶, or R^{6'} represents an aralkyloxycarbonyl group, the aralkyl moiety thereof may be any one of those included within the araliphatic acyl group represented by R³, R⁶, or R^{6'}, but is preferably a benzyloxycarbonyl group.

Where R⁴ and R⁵ represent alkyl groups, they may be the same or different and may be straight or branched chain alkyl groups. They preferably have from 1 to 5 carbon atoms and examples include the methyl, ethyl, propyl, isopropyl, butyl, isobutyl, t-butyl, pentyl, and isopentyl groups.

Where R⁴ and R⁵ represent alkoxy groups, these may be the same or different and may be straight or branched chain groups, preferably having from 1 to 4 carbon atoms. Examples include the methoxy, ethoxy, propoxy, isopropoxy, and butoxy groups. Alternatively, R⁴ and R⁶ may together represent a C₁-C₄ alkylenedioxy group, more preferably a methylenedioxy or ethylenedioxy group.

Preferred classes of compounds of Formula I are as follows:
(1) Compounds in which R³ represents a hydrogen atom, a C₁-C₆ aliphatic acyl group, an aromatic acyl group, or a heterocyclic acyl group.
(2) Compounds in which Y represents an oxygen atom; R¹ and R² are the same or different and each represents a hydrogen atom or a C₁-C₅ alkyl group; R³ represents a hydrogen atom, a C₁-C₆ aliphatic acyl group, an aromatic acyl group, or a pyridinecarbonyl group; and R⁴ and R⁵ are the same or different and each represents a hydrogen atom, a C₁-C₅ alkyl group, or a C₁ or C₂ alkoxy group.
(3) Compounds as defined in (2) above, in which: R¹, R², R⁴, and R⁵ are the same or different and each represents a hydrogen atom or a C₁-C₅ alkyl group; n is 1 or 2; and W represents the -CH₂- or >CO group.
(4) Compounds as defined in (3) above, in which R³ represents a hydrogen atom, a C₁-C₅ aliphatic acyl group, a benzoyl group, or a nicotinyl group.
(5) Compounds as defined in (4) above, in which: R¹ and R⁴ are the same or different and each represents a C₁-C₅ alkyl group; R² and R⁵ are the same or different and each represents the hydrogen atom or the methyl group; and R³ represents a hydrogen atom or a C₁-C₄ aliphatic acyl group.
(6) Compounds in which: W represents the -CH₂- or >CO group; Y and Z both represent oxygen atoms; n is 1 or 2; R¹ and R⁴ are the same or different and each represents a C₁-C₄ alkyl group; R² and R⁵ are the same or different and each represents the hydrogen atom or the methyl group; and R³ represents a hydrogen atom or a C₁-C₄ aliphatic acyl group.
(7) Compounds as defined in (6) above, in which n is 1.
(8) Compounds as defined in (6) or (7) above, in which W represents the -CH₂- group.

Preferred compounds among the compounds of Formula I are those wherein:
R¹ is a C₁-C₄ alkyl group, more preferably a methyl or isobutyl group, most preferably a methyl group;
R² is a hydrogen atom or a C₁-C₄ alkyl group, preferably a hydrogen atom, or a methyl or isopropyl group, more preferably a hydrogen atom or a methyl group, most preferably a methyl group;
R³ is a hydrogen atom, a C₁-C₄ aliphatic acyl group, an aromatic acyl group or a pyridinecarbonyl group, preferably a hydrogen atom, or an acetyl, butyryl, benzoyl, or nicotinyl group, more preferably a hydrogen atom or an acetyl, butyryl or benzoyl group, most preferably a hydrogen atom or an acetyl group;
R⁴ is a hydrogen atom, a C₁-C₄ alkyl group or a C₁ or C₂ alkoxy group, preferably a methyl, isopropyl, t-butyl, or methoxy group, more preferably a methyl or t-butyl group, most preferably a methyl group;
R⁵ is a hydrogen atom, a C₁-C₄ alkyl group or a C₁ or C₂ alkoxy group, preferably a hydrogen atom, or a methyl or methoxy group, more preferably a hydrogen atom or a methyl group, and most preferably a methyl group;
n is 1 or 2, preferably 1;
Y is an oxygen atom;
Z is an oxygen atom or an imino group, most preferably an oxygen atom; and
W is a -CH₂- or >C=O group, preferably a -CH₂- group.

Referring to the general Formula II, the substituents may be any from 1 to 3 selected from nitro, amino, alkylamino, dialkylamino, alkoxy, halo, alkyl, or hydroxy, the aromatic acyl group may be benzoyl and naphthoyl. The alkyl group R₁₁ may be a straight chain or branched alkyl of 1 to 10 carbon atoms, such as methyl, ethyl, n-propyl, i-propyl, n-butyl, i-butyl, t-butyl, n-pentyl, i-pentyl, n-hexyl, n-heptyl, n-octyl, n-nonyl, and n-decyl; the cycloalkyl group R₁₁ may be a cycloalkyl group of 3 to 7 carbon atoms, such as cyclopropyl, cyclopentyl, cyclohexyl, and cycloheptyl; and the phenylalkyl group R₁₁ may be a phenylalkyl group of 7 to 11 carbon atoms such as benzyl and phenethyl. As examples of the heterocyclic group R₁₁ may be mentioned 5- or 6-membered groups each including 1 or 2 hetero-atoms selected from among nitrogen, oxygen, and sulfur, such as pyridyl, thienyl, furyl, thiazolyl, etc. When R₁₁ is 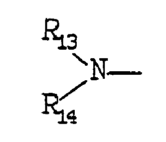 the lower alkyls R₁₃ and R₁₄ may each be a lower alkyl of 1 to 4 carbon atoms, such as methyl, ethyl, n-propyl, i-propyl, and n-butyl. When R₁₃ and R₁₄ are combined to each other to form a 5- or 6-membered heterocyclic group as taken together with the adjacent N atom, i.e., in the form of 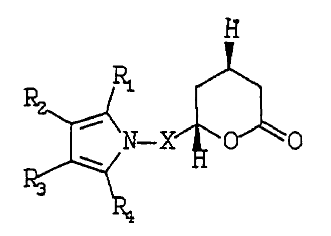 this heterocyclic group may further include a heteroatom selected from among nitrogen, oxygen, and sulfur as exemplified by piperidino, morpholino, pyrrolidino, and piperazino. The lower alkylene group R₁₂ may contain 1 to 3 carbon atoms and thus may be, for example, methylene, ethylene, or trimethylene. The bond R₁₂ is equivalent to the symbol "-", ".", or the like which is used in chemical structural formulas, and when R₁₂ represents such a bond, the compound of general Formula II is represented by the following general Formula II(a) 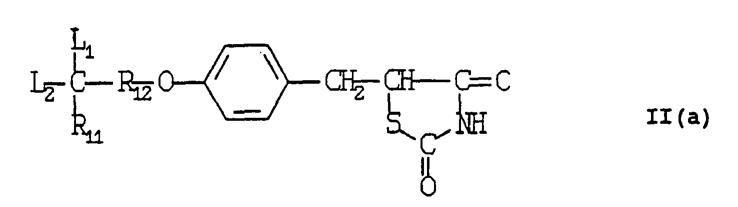 Thus, when R₁₂ is a bond, the atoms adjacent thereto on both sides are directly combined together. As examples of the lower alkyls L₁ and L₂, there may be mentioned lower alkyl groups of 1 to 3 carbon atoms, such as methyl and ethyl. The alkylene group formed as L₁ and L₂ are joined together is a group of the formula -(CH₂)ₙ- [where n is an integer of 2 to 6]. The cycloalkyl, phenylalkyl, phenyl, and heterocyclic groups mentioned above, as well as said heterocyclic group 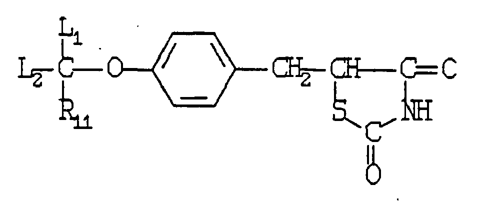 may have 1 to 3 substituents in optional positions on the respective rings. As examples of such substituents may be mentioned lower alkyls (e.g., methyl, ethyl, etc.), lower alkoxy groups (e.g., methoxy, ethoxy, etc.), halogens (e.g., chlorine, bromine, etc.), and hydroxyl. The case also falls within the scope of the general Formula II that an alkylenedioxy group of the formula -O-(CH₂)ₘ-O- [is an integer of 1 to 3], such as methylenedioxy, is attached to the two adjacent carbon atoms on the ring to form an additional ring.

The preferred compounds of Formula III are those wherein R₁₅ and R₁₆ are independently hydrogen, lower alkyl containing 1 to 6 carbon atoms, alkoxy containing 1 to 6 carbon atoms, halogen, ethynyl, nitrile, trifluoromethyl, vinyl, or nitro; n is 1 or 2 and the pharmaceutically acceptable salts thereof.

Preferred in Formula IV are compounds wherein the dotted line represents no bond, particularly wherein D is CO or CHOH. More preferred are compounds wherein V is -CH = CH-, -CH = N- or S and n is 2, particularly those compounds wherein X is O and Y is N, X is S and Y is N, X is S and Y is CH or X is -CH = N- and Y is CH. In the most preferred compounds X is O or S and Y is N forming an oxazol-4-yl, oxazol-5-yl, thiazol-4-yl, or thiazol-5-yl group; most particularly a 2-[(2-thienyl), (2-furyl), phenyl, or substituted phenyl]-5-methyl-4-oxazolyl group.

The preferred compounds in Formula V are:
a) those wherein the dotted line represents no bond, A and B are each CH, X₁ is CO, n is 0, R₁₉ is hydrogen, Z₂ is CH₂CH₂ or CH=CH and X₃ is hydrogen, particularly when X₂ is hydrogen, 2-methoxy, 4-benzyloxy, or 4-phenyl;
b) those wherein A and B are each CH, X₁ is S or SO₂, n is 0, R₁₉ is hydrogen, Z₂ is CH₂CH₂ and X₃ is hydrogen, particularly when X₂ is hydrogen or 4-chloro.

A preferred group of compounds is that of Formula VI wherein R₂₃ is (C₁-C₆)alkyl, (C₃-C₇)cycloalkyl, phenyl, halophenyl, or (C₁-C₆)alkylphenyl. Especially preferred within this group are the compounds where R₂₃ is phenyl, methylphenyl, fluorophenyl, chlorophenyl, or cyclohexyl.

When used herein with regard to Formulas VII through X, the term "aryl" includes phenyl and naphthyl, suitably phenyl, optionally substituted with up to 5, preferably up to 3, groups selected from halogen, alkyl, phenyl, alkoxy, haloalkyl, hydroxy, amino, nitro, carboxy, alkoxycarbonyl, alkoxycarbonylalkyl, alkylcarbonyloxy, or alkylcarbonyl groups.

The term "halogen" refers to fluorine, chlorine, bromine, and iodine; preferably chlorine.

The terms "alkyl" and "alkoxy" relate to groups having straight or branched carbon chains, containing up to 12 carbon atoms.

Suitable alkyl groups are C₁₋₁₂ alkyl groups, especially C₁₋₆ alkyl groups, e.g., methyl, ethyl, n-propyl, iso-propyl, n-butyl, isobutyl, or tert-butyl groups.

Suitable substituents for any alkyl group include those indicated above in relation to the term "aryl".

Suitable substituents for any heterocyclyl group include up to 4 substituents selected from the group consisting of alkyl, alkoxy, aryl, and halogen or any 2 substituents on adjacent carbon atoms, together with the carbon atoms to which they are attached, may form an aryl group, preferably a benzene ring, and wherein the carbon atoms of the aryl group represented by the said 2 substituents may themselves be substituted or unsubstituted.

Specific examples of compounds of the present invention are given in the following list:
(+)-5-[[4-[(3,4-dihydro-6-hydroxy-2,5,7,8-tetramethyl-2H-1-benzopyran-2-yl)methoxy]-phenyl]methyl]-2,4-thiazolidinedione: (troglitazone);
4-(2-naphthylmethyl)-1,2,3,5-oxathiadiazole-2-oxide;
5-[4-[2-[N-(benzoxazol-2-yl)-N-methylamino]ethoxy]-benzyl]-5-methylthiazolidine-2,4-dione;
5-[4-[2-[2,4-dioxo-5-phenylthiazolidin-3-yl)ethoxy]-benzyl]thiazolidine-2,4-dione;
5-[4-[2-[N-methyl-N-(phenoxycarbonyl)amino]ethoxy]-benzyljthiazolidine-2,4-dione;
5-[4-(2-phenoxyethoxy)benzyl]thiazolidine-2,4-dione;
5-[4-[2-(4-chlorophenyl)ethylsulfonyl]benzyl]-thiazolidine-2,4-dione;
5-[4-[3-(5-methyl-2-phenyloxazol-4-yl)propionyl]-benzyl]thiazolidine-2,4-dione;
5-[4-[(1-methylcyclohexyl)methoxy]benzyl]thiadiazolidine-2,4-dione: (ciglitazone);
5-[[4-(3-hydroxy-1-methylcyclohexyl)methoxy]benzyl]-thiadiazolidine-2,4-dione;
5-[4-[2-(5-methyl-2-phenyloxazol-4-yl)ethoxyl]-benzyl]thiadizolidione-2,4-dione;
5-[4-[2-(5-ethylpyridin-2-yl)ethoxyl]benzyl]-thiadiazolidine-2,4- dione: (pioglitazone);
5-[(2-benzyl-2,3-dihydrobenzopyran)-5-ylmethyl]-thiadiazoline-2,4-dione: (englitazone);
5-[[2-(2-naphthylmethyl)benzoxazol]-5-ylmethyl]-thiadiazoline-2,4-dione;
5-[4-[2-(3-phenylureido)ethoxyl]benzyl]thiadiazoline-2,4-dione;
5-[4-[2-[N-(benzoxazol-2-yl)-N-methylamino]ethoxy]-benzy]thiadiazoline-2,4-dione;
5-[4-[3-(5-methyl-2-phenyloxazol-4-yl)propionyl]-benzyl]thiadiazoline-2,4-dione;
5-[2-(5-methyl-2-phenyloxazol-4-ylmethyl)-benzofuran-5-ylmethyl]- oxazolidine-2,4-dione;
5-[4-[2-[N-methyl-N-(2-pyridyl)amino]ethoxy]-benzyl]thiazolidine-2,4-dione; and
5-[4-[2-[N-(benzoxazol-2-yl)-N-methylamino]ethoxy]-benzyl]- oxazolidine-2,4-dione.

As defined herein, "complications of NIDDM" is referred to as cardiovascular complications or several of the metabolic and circulatory disturbances that are associated with hyperglycemia, e.g., insulin resistance, hyperinsulinemia and/or hyperproinsulinemia, delayed insulin release, dyslipidemia, retinopathy, peripheral neuropathy, nephropathy, and hypertension.

The compounds of Formulas I through XIII are capable of further forming pharmaceutically acceptable base salts.

The compounds of Formulas I through XIII are capable of further forming both pharmaceutically acceptable acid addition and/or base salts. All of these forms are within the scope of the present invention.

Pharmaceutically acceptable acid addition salts of the compounds of Formulas I through XIII include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenylsubstituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate, n-methyl glucamine (see, for example, Berge S.M., et al., "Pharmaceutical Salts," Journal of Pharmaceutical Science 1977;66:1-19).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner or as above. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M., et al., "Pharmaceutical Salts," Journal of Pharmaceutical Science 1977;66:1-19).

The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner or as above. The free acid forms differ from their respective salt forms somewhat in certain physical properties such as solubility in polar solvents, but otherwise the salts are equivalent to their respective free acid for purposes of the present invention.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention. Certain of the compounds of the present invention possess one or more chiral centers and each center may exist in different configurations. The compounds can, therefore, form stereoisomers. Although these are all represented herein by a limited number of molecular formulas, the present invention includes the use of both the individual, isolated isomers and mixtures, including racemates, thereof. Where stereospecific synthesis techniques are employed or optically active compounds are employed as starting materials in the preparation of the compounds, individual isomers may be prepared directly; on the other hand, if a mixture of isomers is prepared, the individual isomers may be obtained by conventional resolution techniques, or the mixture may be used as it is, without resolution.

Furthermore, the thiazolidene or oxazolidene part of the compounds of Formulas I through XIII can exist in the form of tautomeric isomers. All of the tautomers are represented by Formulas I through XIII, and are intended to be a part of the present invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form.

The quantity of active component in a unit dose preparation may be varied or adjusted from 0.1 mg to 100 mg preferably 0.5 mg to 100 mg according to the particular application and the potency of the active component. The composition can, if desired, also contain other compatible therapeutic agents.

In therapeutic use in the treatment of at risk populations such as those with impaired glucose tolerance, to prevent or delay the onset of NIDDM and complications arising therefrom, the compounds utilized in the pharmaceutical methods of this invention are administered along with a pharmaceutically acceptable carrier at the initial dosage of about 0.01 mg to about 20 mg per kilogram daily. A daily dose range of about 0.01 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day, if desired.

The compounds of Formulas I through XIII are valuable agents in returning an individual to a state of glucose tolerance and therefore preventing or delaying the onset of NIDDM. The following illustrates testing to show that compounds have the disclosed activity, using the preferred compound troglitazone.

### EXAMPLE 1

In a blinded, randomized, fixed-dose, parallel-group, placebo-controlled, outpatient trial, the effects of the test compound, (+)-5-[[4-[(3,4-dihydro-6-hydroxy-2,5,7,8-tetramethyl-2H-1-benzopyran-2-yl)methoxy]phenyl]-methyl]-2,4-thiazolidinedione (troglitazone), was compared with that of a placebo on glucose tolerance and on insulin sensitivity. The trial in impaired glucose tolerance (IGT) included a 2-week screening period and a 12-week treatment period. Fifty-six patients were randomized to treatment with placebo or 400 mg/day of troglitazone. Oral glucose tolerance tests (OGTT) and frequently sampled intravenous glucose tolerance tests (FSIGTT) to assess insulin sensitivity were performed before study medication, and after 6 weeks and after 12 weeks of randomized treatment.

Patients included in this study were adults in reasonably good health who have IGT by the WHO criteria as demonstrated by OGTT (Harris M.I., Hadden W.C., Knowler W.C., Berrett P.H., International Criteria for the Diagnosis of Diabetes and Impaired Glucose Tolerance, Diabetes Care 1985;8(6):562-7). Most of the patients that were recruited were relatives of patients with NIDDM, patients with a history of gestational diabetes mellitus, patients with a history of prior abnormal OGTT, or patients with other indicators of insulin resistance (coronary artery disease, obesity, hypertriglyceridemia, and hypertension).

The OGTT was carried out according to the following procedure:

Test was administered in the morning after a 10- to 14-hour fast. Water, but not coffee, could be consumed during the fast. Patients were required to remain seated during the test. Study medication was omitted on the morning of the test and taken with lunch.

5 mL of venous blood was collected into a serum separation tube for baseline.

1.75-g/kg body weight, up to a maximum of 75 g of glucose was administered orally as a liquid beverage to be consumed over no more than 5 minutes.

5 mL of venous blood was collected into a serum separation tube every 30 minutes up to 2 hours, timing from the start of ingestion of the glucose.

Each blood specimen was allowed to clot for 30 minutes. The specimens were centrifuged until clot and serum were separated by a well-formed polymer barrier. Serum was transferred from each specimen, using separate pipettes for each, into plastic vials and frozen immediately. If centrifuging of specimens was delayed for any reason, specimens were refrigerated and centrifuged as soon as possible.

Frozen specimens were examined for oral glucose tolerance according to the WHO diagnostic criteria.

**WHO Diagnostic criteria**

| Serum Glucose mg/dL (mmol/L) | Normal | IGT | | Diabetes |
|---|---|---|---|---|
| Fasting | <140 (<7.8) | <140 | (<7.8) | ≥140 (≥7.8) |
| 2 hour | <140 (<7.8) | 140-199 | (7.8-11.1) | ≥200 (≥11.1) |

### PROTOCOL 1

Treatment Effects

| | 2-Hour Glucose (mg/dL) | | Fasting Insulin (UIU/mL) | |
|---|---|---|---|---|
| Test Compound A | Baseline | 6 Week | Baseline | 6 Week |
| 1 | 167.00 | 81.00 | 14.40 | 2.00 |
| 2 | 143.00 | 146.00 | 9.10 | 25.70 |
| 3 | 143.00 | 106.00 | 4.00 | 6.20 |
| 4 | 167.00 | 85.00 | 12.70 | 11.30 |
| 5 | 166.00 | 113.00 | 13.20 | 13.30 |
| 6 | 158.00 | 101.00 | 20.00 | 11.30 |
| 7 | 148.00 | 81.00 | 8.30 | 2.00 |
| 8 | 166.00 | 172.00 | 21.80 | 9.30 |
| 9 | 187.00 | 158.00 | 22.50 | 12.20 |
| 10 | 147.00 | 98.00 | 12.00 | 7.70 |

| Placebo | | | | |
|---|---|---|---|---|
| 1 | 182.00 | 155.00 | 20.70 | 17.20 |
| 2 | 154.00 | 125.00 | 10.30 | 10.90 |
| 3 | 145.00 | 155.00 | 12.30 | 12.10 |
| 4 | 160.00 | 133.00 | 25.90 | 11.60 |
| 5 | 184.00 | 177.00 | 27.70 | 20.20 |
| 6 | 160.00 | 193.00 | 23.90 | 50.30 |
| 7 | 144.00 | 145.00 | 5.50 | 8.60 |
| 8 | 148.00 | 132.00 | 15.40 | 12.20 |
| 9 | 181.00 | 229.00 | 18.10 | 27.70 |
| 10 | 170.00 | 141.00 | 19.80 | 13.50 |

The results of the OGTTs show that treatment with the test compound correlates to reduction of fasting insulin levels and return of glucose tolerance to the normal range for approximately 70% of the subjects. With the exception of one placebo-responder, treatment with placebo does not change significantly the fasting insulin and glucose tolerance profiles.

### PROTOCOL 1

Summary of OGTT Glucose (mg/dL)

| Treatment | Hour | Screening (N = 38) | Week 6 (N = 37) | Week 12 (N = 19) |
|---|---|---|---|---|
| Compound A | 0 | 105 | 88 | 95 |
| | 0.5 | 173 | 153 | 157 |
| | 1.0 | 185 | 151 | 162 |
| | 1.5 | 170 | 145 | 152 |
| | 2.0 | 160 | 123 | 131 |
| Placebo | 0 | 102 | 100 | 99 |
| | 0.5 | 169 | 164 | 163 |
| | 1.0 | 184 | 191 | 186 |
| | 1.5 | 176 | 181 | 176 |
| | 2.0 | 162 | 155 | 150 |

These results show that the average value for 2-hour glucose from the OGTT returns to the normal range for patients treated for 6 weeks and 12 weeks with Test Compound A compared to placebo which shows no significant change in the average value for 2-hour glucose.

### PROTOCOL 1

Conversion After 6 Weeks of Treatment From IGT to Normal by WHO Classification

| Treatment | IGT at Screening | Converted to Normal |
|---|---|---|
| Compound A | 18 | 12 (67%) |
| Placebo | 19 | 7 (37%) |

The results show that on a patient-by-patient analysis, significantly more persons classified with IGT convert to normal glucose tolerance after treatment with Test Compound A (67%) than with placebo (37%).

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. Use of compound of formula (I), as defined below, in the manufacture of a medicament for the treatment of impaired glucose tolerance in order to prevent or delay the onset of noninsulin-dependent diabetes mellitus: 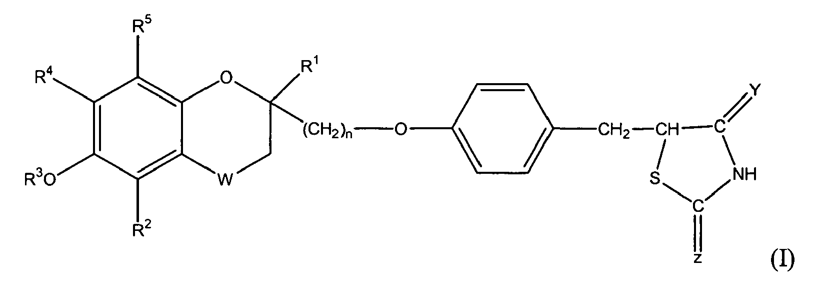 wherein R¹ and R² are the same or different and each represents a hydrogen atom or a C₁-C₅ alkyl group;
R³ represents a hydrogen atom, a C₁-C₆ aliphatic acyl group, an alicyclic acyl group, an aromatic acyl group, a heterocyclic acyl group, an araliphatic acyl group, a (C₁-C₆ alkoxy) carbonyl group, or an aralkyloxycarbonyl group;
R⁴ and R⁵ are the same or different and each represents a hydrogen atom, a C₁-C₅ alkyl group or a C₁-C₅ alkoxy group, or R⁴ and R⁵ together represent a C₁-C₄ alkylenedioxy group; n is 1, 2, or 3;
W represents the -CH₂-, >CO, or CH-OR⁶ group (in which R⁶ represents any 1 of the atoms or groups defined for R³ and may be the same as or different from R³); and
Y and Z are the same or different and each represents an oxygen atom or an imino (=NH) group;
and pharmaceutically acceptable salts thereof.

2. Use according to claim 1 of a compound of formula (I) wherein Y and Z are oxygen.

3. Use according to claim 1 of a compound of formula (I) wherein W is -CH₂-.

4. Use according to claim 1 of a compound of formula (I) wherein n is 1.

5. Use according to claim 1 of a compound of formula (I) wherein R₁, R₂, R₄ and R₅ are lower alkyl and R₃ is H.

6. Use according to claim 1 of a compound of formula (I) wherein Z and Y are oxygen, n is 1, and W is -CH₂-.

7. Use according to claim 1 of a compound of formula (I) wherein the compound is (+)-5-[[4-[(3,4-dihydro-6-hydroxy-2,5,7,8-tetramethyl-2H-1-benzopyran-2-yl)methoxy]phenyl]methyl]-2,4-thiazolidinedione (troglitazone).
